# FASCICULE DE BREVET EUROPEEN

(11) **EP 3 595 582 B1**
(45) Date de publication et mention de la délivrance du brevet: **13.01.2021**
(21) Numéro de dépôt: 18711098.6
(22) Date de dépôt: 14.03.2018
(51) Int. Cl.: A61F 2/16

(54) **DISPOSITIF D'INJECTION DE LENTILLE INTRAOCULAIRE SOUPLE**
VORRICHTUNG ZUM INJIZIEREN EINER FLEXIBLEN INTRAOKULARLINSE
DEVICE FOR INJECTING A FLEXIBLE INTRAOCULAR LENS

(30) Priorité: 15.03.2017 BE 201705159
(43) Date de publication de la demande: 22.01.2020
(73) Titulaire: Physiol S.A., 4031 Angleur (BE)
(72) Inventeur: BOZUKOVA, Dimitriya, 4030 Grivegnée (BE); VERHOYEN, Olivier, 4280 Lens-Saint-Remy (BE); BERTRAND, Tanguy, 4300 Bleret (BE); PAGNOULLE, Christophe, 4800 Verviers (BE)
(74) Mandataire: Gevers Patents
(86) Numéro de dépôt international: PCT/EP2018/056374
(87) Numéro de publication internationale: WO 2018/167140

(56) Documents cités:
- BE-A3- 1 016 692
- US-A1- 2009 171 366
- US-A1- 2011 060 274
- US-A1- 2014 012 277

## Description

### Domaine technique

Selon un premier aspect, l'invention se rapporte à un dispositif d'injection de lentille intraoculaire souple. Selon un deuxième aspect, l'invention se rapporte à une utilisation d'un dispositif d'injection de lentille intraoculaire souple. Selon un troisième aspect, l'invention se rapporte à un procédé d'expulsion d'une lentille intraoculaire souple en-dehors d'un dispositif d'injection.

### Etat de la technique

Les lentilles intraoculaires (LIO) sont destinées au remplacement du cristallin (lentille naturelle) opacifié chez les patients atteints de cataracte, lors d'une intervention chirurgicale. Les interventions de la cataracte sont probablement les opérations les plus pratiquées chez les êtres humains, avec une amélioration significative de la vue chez 98 % des patients. La technique de la chirurgie de la cataracte a accompli, ces dernières années, d'énormes progrès, grâce notamment à la diminution de la taille de l'incision par laquelle le cristallin est extrait et la lentille synthétique implantée dans l'œil. Les nouveaux matériaux souples (silicones, acryliques souples et hydrogels) permettent l'implantation de la lentille par une incision inférieure à celle pratiquée avec les premiers implants rigides en polyméthacrylate de méthyle (PMMA). La lentille souple peut en effet être implantée sous une conformation pliée ou enroulée, avant de recouvrer, une fois mise en place dans l'œil et grâce à ses propriétés viscoélastiques particulières (mémoire de forme), sa forme initiale et définitive. Les petites incisions, sans points de suture, présentent l'avantage de ne pas entraîner de déformation de la cornée et donc de prévenir l'astigmatisme. La récupération de la vue se fait plus rapidement et le résultat réfractif est plus stable.

Avec l'avènement des lentilles souples s'est posé le problème de la pose de ces implants. En effet, pour tirer parti d'une incision réduite, il faut pouvoir plier la lentille et l'introduire sous une forme enroulée dans l'œil. Une première technique connue consiste à implanter la lentille à l'aide d'une pince manuelle. La manipulation de l'implant est cependant fastidieuse. De surcroît, l'incision a dans ce cas une longueur minimale limite (de l'ordre de 3,2 mm) fixée par l'épaisseur des mors de la pince et celle de l'implant plié.

Une technique d'implantation alternative consiste à utiliser un dispositif d'injection adapté aux lentilles souples. Ce dispositif d'injection est généralement constitué de deux éléments distincts : une cartouche (comprenant un canon et une navette parfois appelée navette de stockage), et un corps tubulaire muni d'un poussoir (voir par exemple la demande de brevet BE1016692 (numéro de dépôt : BE2005/0368)). La cartouche permet le pliage, l'enroulement et le déplacement de la lentille enroulée vers un orifice de sortie d'un diamètre réduit au travers duquel l'implant est expulsé dans l'œil, l'injection de la lentille au travers de la cartouche étant assurée par un déplacement du poussoir à la manière d'une seringue. Pour faciliter le déplacement de l'implant dans la cartouche, on fait parfois usage d'un poussoir muni à son extrémité d'un tampon cylindrique qui peut être introduit dans un conduit de la navette de stockage de la cartouche et quelques gouttes d'un additif lubrifiant sont généralement appliquées sur les faces internes de la cartouche. Il s'agit généralement d'un produit visqueux de la famille des polysaccharides (hyaluronate de sodium, cellulose méthylique d'hydroxypropyle, sulfate de chondroïtine...). La technique de l'injection permet l'introduction de l'implant dans des incisions de dimensions pouvant être inférieures à 2 mm, tout en contribuant à une simplification et à une sécurisation de la procédure d'implantation.

En général, une fois la cartouche (pré-chargée avec la lentille) connectée au corps tubulaire de l'injecteur, le praticien procède comme suit.
i) Etape de pré-pliage : le praticien exerce d'abord une première pression avec le pouce sur le poussoir de l'injecteur qui se déplace d'une première position de repos vers une deuxième position d'enroulement de la lentille. Cette dernière est en fait amenée, de par le mouvement du poussoir, de la zone de chargement de la cartouche (appelée navette) où elle se trouve dans une configuration non voire peu déformée vers une zone d'enroulement, dans une partie en aval de la cartouche, dans laquelle la lentille intraoculaire adopte une conformation pré-pliée (cette zone d'enroulement correspond à la partie proximale du canon).
ii) Etape de relâchement (release en anglais): le praticien réalise un ou plusieurs mouvements de recul du poussoir de l'injecteur pour éviter une capture d'haptiques, terme connu d'un homme du métier. Des haptiques accidentellement coincées entre la paroi interne de la cartouche et l'extrémité du poussoir de l'injecteur peuvent ainsi être libérées.
iii) Etape d'injection proprement dite : le praticien pousse à nouveau sur le poussoir de sorte que la lentille soit expulsée hors du canon de la cartouche vers le site d'implantation intraoculaire. Dans cette étape, la lentille est amenée de la zone d'enroulement du canon dans une configuration pré-pliée prenant la forme d'un rouleau vers la zone d'injection (partie distale du canon). La section transversale du canon est en général décroissante depuis la zone d'enroulement vers l'extérieur. Un pliage progressif de la lentille sur elle-même s'opère donc lorsque le poussoir pousse la lentille vers la sortie : au cours du déplacement de la lentille de la zone de chargement vers la zone d'injection et la sortie de cartouche, la lentille passe progressivement d'une configuration pré-pliée vers une configuration enroulée dont le diamètre diminue suite au profil de section interne conique du canon.

Lors de l'étape ii) de relâchement, de l'air peut être aspiré dans la cartouche, via l'orifice de sortie du canon. Cela est possible car la lentille se trouve dans une configuration enroulée et pré-pliée permettant à l'air de passer au travers d'une cavité tubulaire qu'elle définit dans cette position, en communication avec ledit orifice de sortie du canon et le creux de la partie navette de la cartouche. Cet air aspiré vient se loger entre la lentille et le poussoir qui peut être muni a son extrémité distale d'un coussin (en plus de l'air, un additif lubrifiant peut également se loger entre l'extrémité distale du poussoir et la lentille). Lors de l'étape iii) d'éjection, c'est l'air emprisonné qui pousse directement la lentille et pas le poussoir (ou le coussin du poussoir). Cet air peut se comprimer un moment sans induire de mouvement de la lentille vers l'orifice de sortie du canon. Quand une certaine surpression est atteinte, les phénomènes suivants peuvent arriver. Si la pression est suffisante, la cartouche peut exploser. Elle peut aussi éventuellement se désolidariser du corps tubulaire de l'injecteur. Selon une autre possibilité, la surpression a pour effet d'expulser violemment l'implant en-dehors de la cartouche (effet carabine à plomb). Finalement, quand les phénomènes précédents ne se passent pas, la force de résistance du poussoir sur le doigt du praticien est relativement erratique et changeante en fonction du déplacement du poussoir. Cela diminue la qualité du contrôle de l'injection de l'implant dans un œil de patient.

Ainsi, l'utilisation d'un dispositif d'injection tel que décrit dans BE2005/0368 présente des risques d'injection.

US2009/171366 décrit également un dispositif d'injection de lentille intraoculaire souple. Il comprend notamment un corps tubulaire et un poussoir introduit dedans et déplaçable dans la cavité du corps tubulaire. Néanmoins, ce dispositif d'injection présente aussi certains risques ; en particulier, le contrôle de l'injection n'est pas optimal pour les différentes positions du poussoir.

### Résumé de l'invention

Selon un premier aspect, un des buts de la présente invention est de fournir un dispositif d'injection pour lentille intraoculaire souple qui présente moins de risques lors de l'injection de ladite lentille. A cet effet, les inventeurs proposent un dispositif d'injection de lentille intraoculaire souple comprenant :
- un corps tubulaire présentant une enveloppe et une cavité axiale ainsi qu'une extrémité proximale et une extrémité distale,
- un poussoir introduit dans ladite cavité axiale, à l'extrémité proximale susdite, et déplaçable dans cette cavité suivant une direction axiale,
- un canon s'étendant au-delà de ladite extrémité distale du corps tubulaire, présentant une cavité d'éjection ayant une section transversale décroissante, préférentiellement strictement décroissante, en sens opposé au corps tubulaire et délimitée perpendiculairement à ladite direction axiale par une paroi latérale de canon, c'est-à-dire une paroi principale du canon disposée autour de la direction axiale, ledit canon ayant un orifice de sortie (ou extrémité ouverte) pour permettre une sortie d'une lentille intraoculaire souple en-dehors de ladite cavité d'éjection le long de ladite direction axiale,
- de préférence, une navette située entre le canon et ladite extrémité proximale, ayant une cavité de stockage en communication avec ladite cavité d'éjection du canon et la cavité axiale du corps tubulaire pour recevoir une lentille intraoculaire souple, cette cavité de stockage étant délimitée perpendiculairement à ladite direction axiale par une paroi latérale de navette. Le dispositif d'injection selon l'invention est caractérisé en ce que la paroi latérale de canon comprend un (ou est percée d'un) premier trou (ou cavité) traversant situé à une distance comprise entre 2 mm et 10 mm, de préférence entre 4 mm et 8 mm dudit orifice de sortie, et un deuxième trou (ou cavité) traversant situé à une distance comprise entre 6 mm et 10 mm dudit orifice de sortie.. Ces premier et deuxième trous (ou cavités) sont réalisés le long (ou au travers) de l'épaisseur de la paroi latérale de canon. D'une manière équivalente, on pourrait dire que c'est le centre du premier trou qui est situé à une distance comprise entre 2 mm et 10 mm, de préférence entre 4 mm et 8 mm dudit orifice de sortie, et le centre du deuxième trou qui situé à une distance comprise entre 6 mm et 10 mm dudit orifice de sortie. Cette distance peut être mesurée parallèlement à ladite direction axiale ou le long d'une arrête externe de la paroi latérale de canon. Ainsi, ces premier et deuxième trous se trouvent généralement dans une partie du canon qui ne pénètre pas dans l'œil d'un patient.

Après une étape de relâchement ii) mentionnée ci-dessus, et lorsqu'on pousse à nouveau le poussoir lors de l'étape iii), de l'air qui aurait pu être emprisonné entre le poussoir et la lentille intraoculaire souple (ou entre un coussin prolongeant le poussoir et la lentille intraoculaire souple lorsque le dispositif d'injection comprend un tel coussin entre le poussoir et la lentille intraoculaire souple) peut s'échapper par l'intermédiaire des premier et deuxième trous. En l'absence de trou (dispositif d'injection selon l'état de la technique), la lentille intraoculaire souple joue le rôle d'un bouchon empêchant l'air de sortir par l'orifice de sortie du canon.

Le dispositif d'injection de l'invention présente donc les avantages suivants : diminution voire suppression du risque d'explosion ou de décrochage de la cartouche (canon + navette) ou d'une partie de celle-ci ; meilleur contrôle de l'injection car la force à appliquer pour injecter l'implant varie moins le long de la course du poussoir ; suppression de l'effet carabine à plomb. Par la présence des premier et deuxième trous, la résistance mécanique du canon peut être diminuée. Cela permet d'obtenir une plus grande déformation du canon et une moindre déformation de la lentille lorsque cette dernière passe au travers du canon. Cela permet de préserver davantage la lentille intraoculaire à implanter. Ces effets sont d'autant plus intéressants que le relâchement de l'étape ii) est grand, c'est-à-dire que le poussoir est retiré sur une longue course. En effet, en l'absence de trou sur le canon, la quantité d'air emprisonné augmente en général avec la distance parcourue par le poussoir lors de l'étape de relâchement. En outre, la présence des premier et deuxième trous situés à des distances différentes de l'orifice de sortie permet avantageusement d'obtenir l'effet technique souhaité quelque soit la course du poussoir. Ceci est particulièrement avantageux car la course du poussoir est susceptible de dépendre de l'utilisateur du dispositif d'injection. En effet, avec un seul trou, il pourrait être nécessaire de concevoir plusieurs modèles de dispositif d'injection adaptés à chaque utilisateur. Dans le cas de la présente invention, un unique modèle suffit.

Un long relâchement correspond à une course du poussoir vers l'extrémité proximale comprise entre 15 mm et 30 mm. Un relâchement court correspond à une course du poussoir vers l'extrémité proximale comprise entre 3 mm et 14 mm.

Comme cela est illustré dans la description de certains modes de réalisation, l'invention peut être implémentée avec différents types de dispositif d'injection ou injecteur. Selon un exemple possible la navette est configurée de sorte qu'il est possible de l'ouvrir pour accéder à la cavité de stockage et y déposer une lentille intraoculaire souple. Dans d'autres exemples décrits plus bas, il n'est pas possible d'ouvrir la navette sans l'endommager.

L'ensemble canon plus navette est parfois appelé cartouche par un homme du métier. Les trous pratiqués dans le canon (et de préférence dans la deuxième partie de la paroi latérale de canon) ne sont pas nécessairement réalisés à l'apex, selon une verticale. De préférence, ils sont réalisés dans une région comprise entre -30° et +30° par rapport à cette direction verticale.

Comme les premier et deuxième trous sont traversants, il y a une communication entre la cavité d'éjection et l'extérieur du canon quelle que soit la pression à l'intérieur du canon. De préférence, dans le cadre de ce document, un trou traversant d'une paroi est une cavité apte à contenir un espace en forme de cylindre allongé symétriquement le long d'un axe et tronqué de part et d'autre de cet axe, cet espace traversant la paroi, et une section de cet espace selon un plan transverse à cet axe correspondant essentiellement à une section du trou selon ce plan. En particulier, les premier et deuxième trous sont préférentiellement des cavités aptes à contenir chacune un espace en forme de cylindre allongé symétriquement le long d'un axe et tronqué de part et d'autre de cet axe, cet espace traversant la deuxième partie de la paroi latérale du canon, et une section de cet espace selon un plan transverse à cet axe correspondant essentiellement à une section du trou selon ce plan. Préférentiellement, l'axe est perpendiculaire à la direction axiale. Les premier et deuxième trous sont ainsi avantageusement isotropes et au moins partiellement symétriques autour de l'axe, de telle façon à ce que le canon soit plus résistant aux contraintes techniques d'utilisation du dispositif d'injection. De tels premier et deuxième trous se distinguent donc avantageusement d'une ou plusieurs fentes ne satisfaisant pas la définition préférée d'un trou et étant susceptibles de fragiliser le canon ou d'engendre sa rupture lors de l'utilisation du dispositif d'injection. Dans le présent document, un cylindre compris dans une forme d'un trou est nécessairement un cylindre fini.

Un dérivé de l'invention pourrait proposer un premier et/ou un deuxième trou qui serait non traversant de telle façon qu'il existerait alors une membrane d'épaisseur comprise entre 1 et 100 µm (de préférence entre 20 et 40 µm) empêchant une communication entre la cavité d'éjection et l'extérieur du canon pour une pression atmosphérique normale à l'intérieur du canon. De préférence, cette membrane se situerait au niveau de la cavité d'éjection plutôt qu'au niveau du côté externe de la paroi latérale de canon. Cette membrane serait faite de préférence du même matériau que la paroi latérale de canon. De préférence, ce matériau serait du polypropylène. Ainsi, lorsqu'une surpression serait atteinte dans la cavité d'éjection du canon par exemple, lors de l'utilisation du dispositif d'injection, la membrane se déchirerait et permet à de l'air emprisonné de s'échapper, de telle façon à ce la configuration du dispositif d'injection se ramène, à cet instant, à celle du dispositif d'injection selon la présente invention.

Différentes formes pour le premier trou et/ou le deuxième trou peuvent être envisagées. De préférence, il présente une symétrie de révolution autour d'un axe de premier et/ou deuxième trou. Cet axe de premier et/ou deuxième trou est de préférence orienté par rapport à la direction axiale de sorte qu'ils forment ensemble un angle compris entre 85° et 95°. De manière encore préférée, cet axe de premier et/ou deuxième trou est essentiellement perpendiculaire à ladite direction axiale. Avoir de telles orientations pour l'axe de premier et/ou deuxième trou permet de réduire les surface et arêtes vives avec lesquelles la lentille intraoculaire souple peut entrer en contact. Au final, de telles orientations de l'axe de premier et/ou deuxième trou permettent donc de réduire les risques d'endommagement de la lentille lors de sa progression dans le canon. Prévoir de telles orientations pour l'axe de premier et/ou deuxième trou permet également d'avoir une méthode de fabrication de ce trou plus simple. Avoir un ou deux trous ayant une symétrie de révolution par rapport à un axe permet également d'avoir une méthode pour le fabriquer plus simple. Selon un mode de réalisation préféré, le premier et/ou deuxième trou a la forme d'un tronc de cône. Selon un autre mode de réalisation préféré, le premier et/ou deuxième trou a une forme comprenant un ou plusieurs cylindres qui sont préférentiellement coaxiaux et/ou tronqués selon un plan sécant à un axe de symétrie des cylindres qui est perpendiculaire à la direction axiale. Préférentiellement, un mode de réalisation plus préféré de ce mode de réalisation préféré est tel que le premier et/ou deuxième trou ont la forme d'une superposition d'au moins deux cylindres comprenant, de façon préférentielle, un premier cylindre d'un diamètre compris entre 0.7 et 0.5 mm, et un deuxième cylindre d'un diamètre compris entre 0.4 et 0.2 mm, le premier cylindre surmontant le deuxième cylindre. Les premiers et/ou deuxièmes et/ou autres cylindres sont préférentiellement tronqués selon un plan sécant à l'axe de symétrie des cylindres.

De préférence, le premier et/ou deuxième trou présente une extension minimale le long de ladite direction axiale comprise entre 0.3 mm et 0.5 mm. Selon un mode de réalisation, cette extension minimale vaut 0.4 mm. Lorsque le premier et/ou deuxième trou a la forme d'un tronc de cône, cette extension minimale est de préférence située au plus proche de la cavité d'éjection du canon plutôt qu'au niveau de l'extérieur du canon. Lorsque le premier et/ou deuxième trou a la forme d'un tronc de cône, l'extension maximale du premier et/ou deuxième trou mesurée le long de la direction axiale est de préférence comprise entre 0.6 mm à 0.8 mm, avec une valeur particulière possible de 0.7 mm. D'autres dimensions du ou des trous sont possibles. On pourrait dire que le ou les trous ont de préférence un diamètre nominal ou diamètre moyen le long de l'épaisseur du canon égal à 0.4 mm.

Selon un mode de réalisation préféré, le canon comprend plusieurs trous. De préférence, il comprend un premier et un deuxième trous :
- ledit premier trou étant situé à 6 mm de l'orifice de sortie, et
- ledit deuxième trou étant situé à 8 mm de l'orifice de sortie.
C'est la paroi latérale de canon qui est alors percée de ces premier et deuxième trous. De préférence, ces premier et deuxième trous sont situés le long d'une droite sécante à l'axe principal du dispositif d'injection.

Les différentes variantes présentées ci-dessus pour le premier et/ou le deuxième trou s'appliquent au cas où il y a un nombre quelconque de trous. Les avantages associés s'appliquent, mutatis mutandis.

En particulier, selon une variante possible, les premier et deuxième trous présentent chacun une symétrie de révolution autour d'un axe de premier et deuxième trou qui sont essentiellement perpendiculaires à ladite direction axiale. Il est également possible de prévoir un mode de réalisation où les axes de premier et deuxième trou définissent avec la direction axiale un angle compris entre 85° et 95°.
Selon une variante possible, les premier et deuxième trous ont la forme d'un tronc de cône ou une forme s'y rapprochant. Selon une autre variante possible, les premier et deuxième trous ont une forme d'une superposition d'au moins un cylindre, préférentiellement d'au moins deux cylindres.

De préférence, les premier et deuxième trous présentent une extension minimale parallèlement à ladite direction axiale comprise entre 0.3 mm et 0.5 mm, de préférence égale à 0.4 mm.

En général, les parois latérales de canon et de navette présentent chacune une partie (de préférence moitié) inférieure pour entrer en contact avec une lentille intraoculaire souple repliée et une partie (de préférence moitié) supérieure pour entrer en moindre contact avec ladite lentille intraoculaire souple repliée, quand lesdits canon et navette contiennent ladite lentille intraoculaire souple. Les premier et deuxième trous sont pratiqués sur (ou dans) la deuxième partie de la paroi latérale de canon. Cela permet de s'assurer que de l'air emprisonné peut en effet s'échapper par les trous, sans qu'il y ait bouchage des trous par la lentille intraoculaire souple contenue dans le canon. La première partie de la paroi latérale de canon ne comprend pas de trou : seule la deuxième partie de la paroi latérale de canon comprend plusieurs trous. Cela permet de réduire le risque d'endommagement de la lentille intraoculaire souple : comme elle est davantage en contact avec la première partie de la paroi latérale de canon, il y a moins de risque d'endommager la lentille si ladite première partie de la paroi latérale de canon ne comprend pas de trou. De préférence, la première (respectivement deuxième) partie de la paroi latérale de canon est une partie inférieure (respectivement supérieure).

Les première et deuxième parties des parois latérales de canon et de navette sont en prolongement l'une de l'autre. Elles sont situées d'un même côté de l'axe principal du dispositif d'injection qui est parallèle à la direction axiale. Dans un mode de réalisation préféré, la deuxième partie de la paroi latérale de navette comprend une excroissance ou ailette qui peut venir s'insérer dans une fente pratiquée dans l'enveloppe du corps tubulaire.

Les première et deuxième parties de surface latérale de canon et de navette peuvent être définies en général par rapport à l'œil d'un patient lors de l'injection d'un implant pratiquée par un chirurgien utilisant le dispositif d'injection de l'invention. Dans ce cas, lesdites deuxièmes parties sont généralement situées du côté de la cornée, tandis que lesdites premières parties inférieures sont situées vers le fond d'œil (ou intérieur de l'œil). Dans ce cas, les deuxièmes parties sont généralement situées vers le haut tandis que les premières parties sont généralement orientées vers le bas, quand le patient est typiquement en position couchée.
Un autre positionnement (ou une autre orientation) des première et deuxième parties de surface latérale de canon et de navette est possible, par exemple pour certaines interventions spécifiques.

De préférence, la première partie de la paroi latérale de canon ne comprend pas de trou. Cette partie est donc de préférence intègre, avec une épaisseur essentiellement constante dans une section droite perpendiculaire à la direction axiale. Dans ce cas, les risques d'abîmer la lentille intraoculaire sont réduits d'avantage. En effet, cette dernière est plus en contact avec la première partie de la paroi latérale de canon. Un trou dans cette partie peut présenter des arêtes qui peuvent abîmer la lentille intraoculaire lorsqu'elle se déplace parallèlement à la direction axiale.
De préférence, la première partie de la paroi latérale de canon représente essentiellement une moitié de la paroi latérale de canon.

Selon un mode de réalisation particulier de l'invention, le canon comprend une portion distale et une portion proximale, la portion distale comprenant l'orifice de sortie, la portion proximale étant comprise entre la portion distale et l'extrémité distale du corps tubulaire ; une épaisseur de la paroi latérale de canon de la portion distale du canon, définie perpendiculairement à la direction axiale, étant strictement plus petite qu'une épaisseur de la paroi latérale de la portion proximale du canon définie également perpendiculairement à la direction axiale ; et les premier et deuxième trous sont situés dans la portion proximale du canon.
Avantageusement, les premier et deuxième trous sont alors situés dans une portion plus épaisse de la paroi latérale du canon, ce qui garantit l'intégrité physique du canon lors de sont utilisation et évite tout risque de rupture du canon lors de l'utilisation du dispositif d'injection.
Préférentiellement, seule la portion distale du canon est apte à pénétrer dans l'œil d'un patient.
Préférentiellement, l'épaisseur de la paroi latérale de canon est définie par une mesure de longueur perpendiculairement à la direction axiale. L'épaisseur de ladite paroi latérale de canon de ladite portion distale est préférentiellement essentiellement constante et comprise entre 0,1 mm et 0,3 mm. L'épaisseur de ladite paroi latérale de canon de ladite portion proximale est préférentiellement essentiellement constante et comprise entre 0,6 mm et 0,9 mm.
Ce mode de réalisation n'est pas limitatif du cas où au moins un des premier et deuxième trous est situé dans la portion distale du canon.

De préférence, le canon est biseauté au niveau de l'orifice de sortie. Dans ce cas, la deuxième partie (de préférence deuxième moitié) du canon correspond à la partie la plus longue de la paroi latérale de canon parallèlement à la direction axiale, tandis que la première partie (de préférence première moitié) du canon correspond à la partie la plus courte de la paroi latérale de canon parallèlement à la direction axiale. Cela permet une implantation sécurisée d'une lentille intraoculaire dans un sac capsulaire. L'inverse est cependant possible. Donc, dans ce denier cas et si le canon est biseauté, la première partie (de préférence première moitié) du canon correspond de préférence à la partie la plus longue de la paroi latérale de canon parallèlement à la direction axiale, tandis que la deuxième partie (de préférence deuxième moitié) du canon correspond de préférence à la partie la plus courte de la paroi latérale de canon parallèlement à la direction axiale. Dans le cas d'un canon biseauté, une seule partie de la paroi latérale de canon comprend un ou plusieurs trous : la deuxième partie.

Selon un mode de réalisation possible, canon et navette font partie d'une même pièce appelée cartouche. Dans ce cas, la cartouche peut être fabriquée d'un seul tenant ou au contraire être obtenue par soudage d'un canon avec une navette.

Dans un autre mode de réalisation, canon et navette sont deux pièces séparées. Dans ce cas, le canon est de préférence solidaire du corps tubulaire tandis que la navette peut être stockée indépendamment du corps tubulaire comprenant le canon. Dans ce mode de réalisation, le dispositif d'injection est de préférence tel qu'il comprend :
- un corps tubulaire présentant une enveloppe et une cavité axiale ainsi qu'une extrémité proximale et une extrémité distale,
- un poussoir introduit dans ladite cavité axiale, à l'extrémité proximale susdite, et déplaçable dans cette cavité suivant une direction axiale,
- un canon agencé à ladite extrémité distale du corps tubulaire et présentant une cavité d'éjection en communication avec la cavité axiale du corps tubulaire, cette cavité d'éjection ayant une section transversale décroissante en sens opposé au corps tubulaire jusqu'à déboucher sur un orifice de sortie du canon, et
- un logement dans la cavité axiale du corps tubulaire destiné à recevoir une navette de stockage contenant une lentille intraoculaire souple,
l'enveloppe du corps tubulaire présentant une ouverture latérale, qui donne accès de l'extérieur audit logement, de manière à permettre une introduction de la navette de stockage dans celui-ci, et en ce que, l'enveloppe susdite porte extérieurement en saillie un premier moyen de fixation rotative capable de coopérer avec un deuxième moyen de fixation rotative prévu sur la navette de stockage, de façon à permettre, en position de coopération des deux moyens de fixation rotative susdits, une rotation de la navette de stockage autour d'un axe de rotation parallèle à ladite direction axiale de la cavité axiale du corps tubulaire, entre une première position de la navette de stockage où elle est à l'extérieur du corps tubulaire et une deuxième position de la navette de stockage où elle est chargée dans le logement susdit ;
le canon comprenant un premier trou traversant situé à une distance comprise entre 4 mm et 8 mm dudit orifice de sortie, et un deuxième trou traversant situé à une distance comprise entre 6 mm et 10 mm dudit orifice de sortie.

Pour l'ensemble des modes de réalisation possibles, différents types de matériaux connus d'un homme du métier peuvent être utilisés pour réaliser le canon et/ou la navette. Des exemples de matériaux sont : polypropylène, PA11 (polyamide 11) ou copolymères de type Vestamid.

Le poussoir est avantageusement muni d'un coussin en matière souple à son extrémité distale pour pousser une lentille intraoculaire souple sans l'endommager. Dans ce mode de réalisation préféré, de l'air pourrait être emprisonné entre ce coussin et une lentille intraoculaire souple en l'absence du premier et/ou deuxième trous.
Selon un autre mode de réalisation de l'invention, le poussoir est muni d'un manchon à son extrémité distale pour pousser une lentille intraoculaire souple. Le manchon est solidaire du poussoir. Il est apte à se déformer pour pousser la lentille intraoculaire souple. Le manchon est préférentiellement composé au moins partiellement de silicone.
Selon un mode de réalisation de l'invention, le poussoir est muni d'un manchon solidaire du poussoir et/ou d'un coussin en matière souple à son extrémité distale pour pousser une lentille intraoculaire souple.

Selon un mode de réalisation possible, le dispositif d'injection de l'invention comprend une lentille intraoculaire souple, dans la navette ou dans le canon. Dans ce cas, la lentille intraoculaire souple est généralement en contact avec une première partie de paroi latérale de canon ou de navette et en moindre contact avec une deuxième partie de paroi latérale de canon ou de navette.

Les trous pratiqués dans la deuxième partie de la paroi latérale de canon ne sont pas nécessairement positionnés selon l'apex, le long d'une direction verticale. Il est possible par exemple de les réaliser sur une portion de surface latérale de canon comprise entre -30° et +30° par rapport à une telle direction verticale. Première et deuxième parties de la surface latérale de canon (respectivement de navette) forment ensemble l'entièreté de la surface latérale de canon (respectivement de navette).

Selon un deuxième aspect, les inventeurs proposent d'utiliser le dispositif d'injection du premier aspect de l'invention pour libérer au travers du premier et/ou deuxième trous de l'air emprisonné entre le poussoir et une lentille intraoculaire souple contenue (ou placée) dans le dispositif d'injection, lors d'un déplacement du poussoir le long de la direction axiale vers ladite extrémité distale. L'air peut s'évacuer par l'intermédiaire des premier et/ou deuxième trous qui sont à l'origine traversant.

Selon un troisième aspect, les inventeurs proposent un procédé d'expulsion d'une lentille intraoculaire souple en-dehors d'un dispositif d'injection et comprenant les étapes suivantes :
- fournir un dispositif d'injection comprenant une lentille intraoculaire souple dans la navette, la lentille intraoculaire souple étant en contact avec la première partie de la paroi latérale de navette et en moindre contact avec la deuxième partie de la paroi latérale de navette ;
- pousser le poussoir le long de ladite direction axiale vers l'extrémité distale pour diriger la lentille intraoculaire souple dans le canon de sorte qu'elle soit en contact avec la première partie de la paroi latérale de canon et en moindre contact avec la deuxième partie de la paroi latérale de canon;
- retirer le poussoir le long de ladite direction axiale vers l'extrémité proximale de sorte que de l'air entre via l'orifice de sortie du canon et vienne se loger entre ledit poussoir et ladite lentille intraoculaire souple présente dans le dispositif d'injection;
- pousser à nouveau le poussoir le long de ladite direction axiale vers l'extrémité distale pour libérer l'air emprisonné à l'étape précédente au travers du premier et/ou deuxième trous (une fois que la lentille intraoculaire souple est située entre l'orifice de sortie du canon et le premier ou deuxième trou).
Généralement, la lentille intraoculaire souple est repliée ou enroulée de sorte que le ou les trous ne soient pas bouchés ou pas entièrement bouchés par elle. Cela est possible car la lentille intraoculaire souple est en contact avec les premières parties de navette et de canon et en moindre contact avec les deuxièmes parties de navette et de canon, vu la configuration repliée ou enroulée de la lentille.
De préférence, le procédé suivant le troisième aspect de l'invention est implémenté (ou réalisé) lorsque le canon est en-dehors d'un mammifère, en particulier, en-dehors d'un corps humain vivant, en particulier en dehors d'un œil.

Les avantages associés au dispositif de l'invention s'appliquent à l'utilisation ou la méthode selon les deuxième et troisième aspects de l'invention, mutatis mutandis. L'objet de ces aspects de l'invention peut être avantageusement utilisé pour réaliser des tests non médicaux, en particulier des tests d'éjections de lentilles intraoculaires sur la résistance des lentilles intraoculaires lors de leurs éjections et/ou sur la résistance du dispositif d'injection selon l'invention. Ces tests permettent avantageusement une plus grande sécurité d'utilisation de lentilles intraoculaires et/ou de dispositifs d'injection selon l'invention lors d'interventions médicales.

### Brève description des figures

Ces aspects ainsi que d'autres aspects de l'invention seront clarifiés dans la description détaillée de modes de réalisation particuliers de l'invention, référence étant faite aux dessins des figures, dans lesquelles:
- la Fig.1 montre un mode de réalisation du dispositif d'injection de l'invention;
- les Fig.2-4 montrent des coupes longitudinales du dispositif d'injection de l'invention selon un mode de réalisation possible;
- la Fig.5 montre une réalisation possible d'une cartouche ;
- les Fig.6-7 montrent des sections droites de réalisations possibles d'une navette et d'un canon respectivement;
- la Fig.8 montre un autre mode de réalisation du dispositif d'injection de l'invention;
- les Fig.9A et 9B montrent des exemples de premiers et deuxièmes trous traversant;
- la Fig.10 montre un exemple d'un premier et deuxième trous non traversant ;
- les Fig.11 et 12 montrent deux sections transversales du canon à deux endroits différents le long de la direction axiale ;
- la Fig. 13 montre une section droite longitudinale d'un canon biseauté.
Les dessins des figures ne sont pas à l'échelle. Généralement, des éléments semblables sont dénotés par des références identiques dans les figures. La présence de numéros de référence aux dessins ne peut être considérée comme limitative, y compris lorsque ces numéros sont indiqués dans les revendications.

### Description détaillée de certains modes de réalisation de l'invention

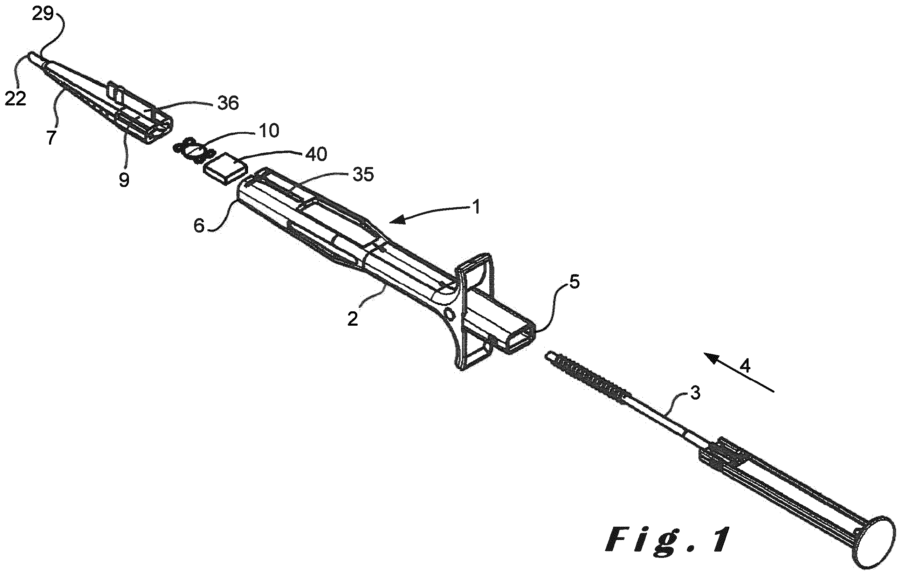

La figure 1 montre un exemple de mode de réalisation du dispositif d'injection selon l'invention. Le long d'une direction axiale 4, il comprend : un poussoir 3 introduit dans la cavité axiale d'un corps tubulaire 1, au niveau de son extrémité proximale 5. Ce poussoir 3 peut être prolongé d'un coussin 40 déformable pour entrer en contact avec une lentille intraoculaire souple 10 sans l'abîmer. Ce mode de réalisation n'est pas limitatif d'un mode de réalisation non représenté où le coussin serait remplacé par un manchon en silicone solidaire du poussoir et apte à se déformer pour pousser la lentille intraoculaire souple. Le coussin 40 déformable possède de préférence un facteur de compressibilité très élevé. De préférence, une section transversale du coussin 40 peut être réduite d'un facteur supérieur à deux, lorsque le coussin 40 est déplacé sous l'action du poussoir 3 de la navette 9 vers l'orifice de sortie 22 du canon 7. De préférence, le coussin 40 est constitué d'une mousse ou d'un élastomère non expansé. Cela est connu de BE101669 (ou BE2005/0368) dont toutes les caractéristiques du coussin 40 sont intégrées ici par référence, pour des modes de réalisation préférés comprenant un tel coussin 40.

La lentille intraoculaire souple 10 est au départ contenue dans la navette 9 d'une cartouche, la navette 9 comprenant une cavité de stockage dans laquelle le coussin 40 et le poussoir 3 peuvent pénétrer. La cartouche comprend également un canon 7 ayant une cavité d'éjection de section transversale décroissante lorsqu'on s'éloigne du corps tubulaire 1. Le canon 7 comprend un orifice de sortie 22 pour permettre à la lentille intraoculaire souple 10 de sortir suite à l'action du poussoir 3 sur la lentille intraoculaire souple 10. Comme on peut le voir sur la figure 1, la cartouche comprend de préférence une ailette 36 qui peut être introduite dans une fente 35 pratiquée sur l'enveloppe 2 du corps tubulaire 1.Cela assure un bon et unique positionnement de la cartouche (et donc de la navette 9 et du canon 7) sur le corps tubulaire 1 et un bon pinçage des deux éléments entre eux. Une fois la cartouche introduite sur le corps tubulaire 1 montré à la figure 1, ce dernier est prolongé au niveau de l'extrémité distale 6 du canon 7.

De préférence, la cartouche de l'exemple illustré à la figure 1 est stockée dans un récipient contenant un liquide avant son usage. Cela est connu d'un homme du métier. Le corps tubulaire 1 est de préférence réalisé dans une matière plastique. La longueur du corps tubulaire 1 le long de la direction axiale 4, entre ses extrémités proximale 5 et distale 6, est de préférence comprise entre 7.5 et 12 cm, avec une valeur encore préférée de 9.5 cm.

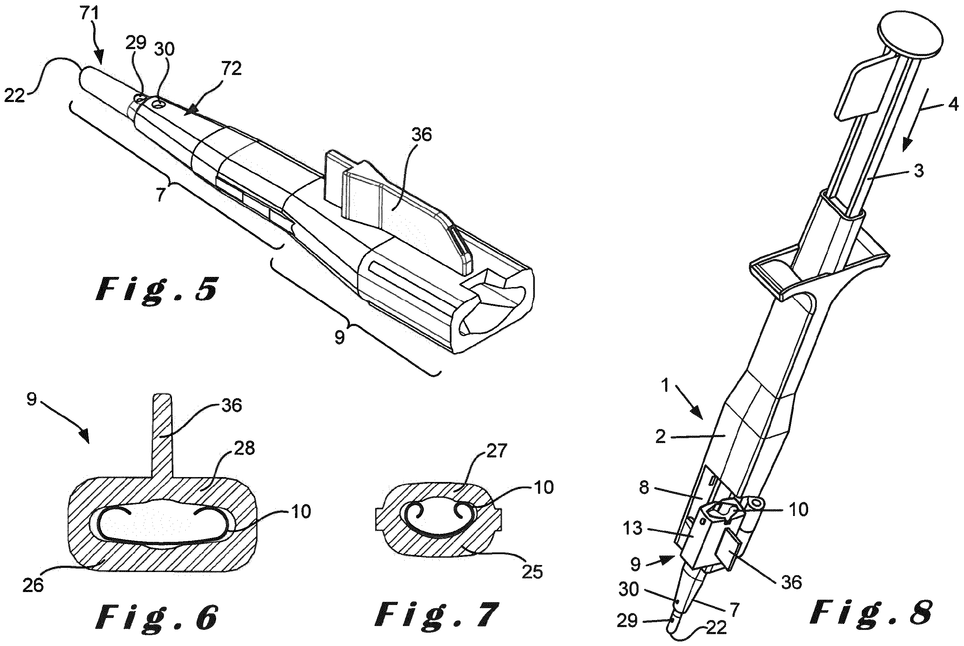

Dans l'exemple montré à la figure 1, le canon 7 comprend au niveau d'une deuxième partie 27 de sa paroi latérale un premier trou 29. Ce trou 29 peut être traversant ou non traversant. De préférence, la distance entre ce premier trou 29 et l'orifice de sortie 22 du canon 7 est comprise entre 4 et 8 mm. La figure 7 illustre la position de la deuxième partie 27 de la paroi latérale de canon.

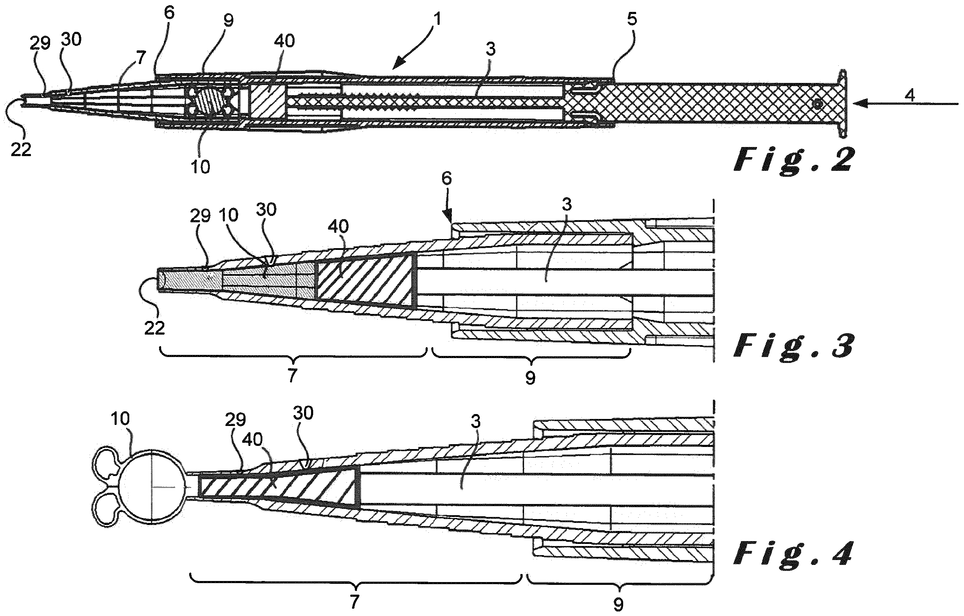

Les figures 2 et 3 montrent une section transversale du dispositif d'injection de la figure 1 lorsque le canon 7 comprend un premier 29 et un deuxième 30 trous. A la figure 3 (respectivement 4), le poussoir 3 est d'avantage introduit dans le corps tubulaire 1 par rapport à la figure 2 (respectivement 3), poussant la lentille intraoculaire souple 10 vers l'orifice de sortie du canon 22. A la figure 4, la lentille intraoculaire souple 10 est presque entièrement sortie. Comme on peut le voir aux figures 3 et 4, il n'y a pas d'air emprisonné entre le coussin 40 et la lentille intraoculaire souple 10 grâce à l'existence des trous (29,30) qui permettent de l'évacuer. L'absence d'air emprisonné est également possible si aucun coussin 40 n'est utilisé entre le poussoir 3 et la lentille intraoculaire souple 10.

La figure 5 montre une vue en perspective d'une cartouche comprenant un canon 7 et une navette 9. Dans cet exemple, canon 7 et navette 9 font donc partie d'une seule et même pièce. Dans l'exemple illustré à la figure 5, le canon 7 comprend deux trous (29,30). Le canon comprend une portion distale 71 et une portion proximale 72, la portion distale 71 comprenant l'orifice de sortie 22, la portion proximale 72 étant comprise entre la portion distale 71 et l'extrémité distale 6 du corps tubulaire 1. L'épaisseur de la paroi latérale sur la portion distale 71 est plus petite que l'épaisseur de la paroi latérale sur la portion proximale 72.

Les figures 6 et 7 montrent des sections droites de la navette 9 et du canon 7 respectivement, lorsqu'ils contiennent une lentille intraoculaire souple 10. Comme on peut le voir sur cette figure, les parois latérales de navette et de canon comprennent chacune une première partie (25,26) et une deuxième partie (27,28). Chaque deuxième partie (27,28) entre en moindre contact avec la lentille intraoculaire souple 10 vu la façon dont elle est repliée en général. C'est pourquoi les inventeurs proposent de réaliser le ou les trous (29,30) sur la deuxième partie 27 de canon 7. Ainsi, on réduit le risque que la lentille intraoculaire souple 10 bouche un trou (29,30) lors de son déplacement vers l'orifice de sortie 22. Les parties supérieures (27,28) (respectivement inférieures (25,26)) du canon 7 et de la navette 9 sont dans un même prolongement. Comme on peut le voir à la figure 6, la deuxième partie 28 de navette 9 comprend de préférence une ailette 36.

La figure 8 montre un autre mode de réalisation possible du dispositif d'injection selon l'invention. Dans ce mode de réalisation, canon 7 et navette 9 sont deux éléments distincts. Le canon 7 prolonge le corps tubulaire 1 à son extrémité distale 6 tandis que la navette 9 (contenant au départ une lentille intraoculaire souple 10) peut venir se placer dans un logement 8 pratiqué dans le corps tubulaire 1. Dans le mode de réalisation illustré à la figure 8, des moyens de fixation rotative présents sur le corps tubulaire 1 et sur la navette 9 permettent de les coupler mécaniquement de manière rotative. Cela est décrit en détails dans la demande BE2016/5271 dont l'entièreté est intégrée ici par référence. Le canon 7 comprend un ou plusieurs trous, de préférence deux trous (29,30) comme cela est illustré à la figure 8. De préférence, le premier (respectivement deuxième) trou 29 (respectivement (30)) est situé à une distance de l'orifice de sortie 22 comprise entre 4 mm et 7 mm (respectivement entre 6 mm et 10 mm), avec une valeur encore préférée de 6 mm (respectivement 8 mm).

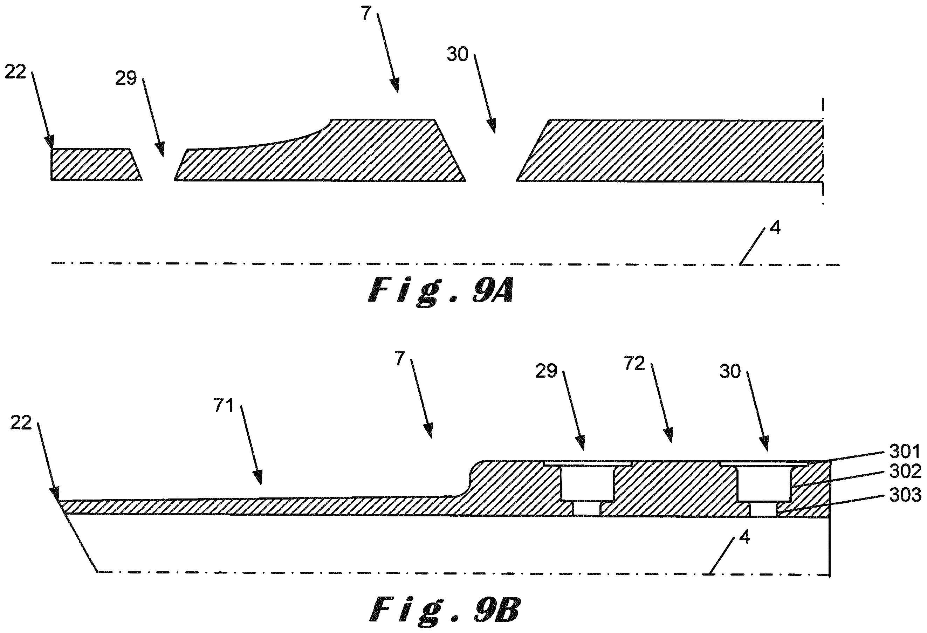

Le ou les trous (29,30) peuvent être traversant ou non traversant, mais sont préférentiellement traversant. Les figures 9A et 9B montrent une vue rapprochée d'une section droite d'un canon 7 selon l'invention comprenant un premier 29 et un deuxième 30 trous traversant. Avec ce mode de réalisation, il y a une communication directe entre l'intérieur du canon 7 et l'extérieur quelle que soit la valeur de la pression à l'intérieur du canon 7. De préférence, le ou les trous (29 ;30) ont la forme d'un tronc de cône, ou s'en rapprochent (ce mode de réalisation est illustré en figure 9A) ou ont la forme d'une superposition de cylindres tronqués, ou s'en rapprochent (ce mode de réalisation est illustré en figure 9B). L'axe de symétrie est alors de préférence orienté à 90° par rapport à la direction axiale 4. Cela évite d'avoir des arêtes vives qui pourraient abîmer la lentille intraoculaire souple 10 quand elle se déplace vers l'orifice de sortie 22. De préférence, l'extension minimale du premier et deuxième trous (29 ; 30) le long de (ou parallèlement à) la direction axiale 4 est compris entre 0.3 mm à 0.5 mm, avec une valeur encore préférée de 0.4 mm. De préférence, l'extension maximale du premier et deuxième trous (29 ; 30) le long de (ou parallèlement à) la direction axiale 4 est compris entre 0.6 mm à 0.8 mm, avec une valeur encore préférée de 0.7 mm. Le deuxième trou 30 illustré en figure 9B a une forme d'une superposition de trois cylindres comprenant un premier cylindre 301 d'un diamètre compris entre 1 et 0.8 mm et d'une hauteur comprise entre 0.1 mm et 0 mm, un deuxième cylindre 302 d'un diamètre compris entre 0.7 et 0.5 mm et d'une hauteur comprise entre 0.4 mm et 0.6 mm, et un troisième cylindre 303 d'un diamètre compris entre 0.4 et 0.2 mm et d'une hauteur comprise entre 0.1 mm et 0.3 mm. Lorsque le premier cylindre 301 est de hauteur nulle, le mode de réalisation se limite au cas particulier où le deuxième trou 30 a la forme d'une superposition de deux cylindres. En d'autres termes, le premier cylindre 301 est optionnel dans ce mode de réalisation particulier de l'invention. Préférentiellement, tel qu'illustré en figure 9B, le premier cylindre surmonte le deuxième cylindre qui surmonte lui-même le troisième cylindre. Préférentiellement, tous les cylindres ont le même axe de symétrie et chaque cylindre est tronqué perpendiculaire à cet axe de symétrie commun, ce dernier étant perpendiculaire à la direction axiale 4. La figure 9B n'est pas limitative d'un cas où les cylindres seraient tronqués obliquement par rapport à des axes de symétries différents. Les premier 29 et deuxième 30 trous illustrés en figure 9B ont la même forme. Ils sont situés dans la portion proximale 72 du canon 7.

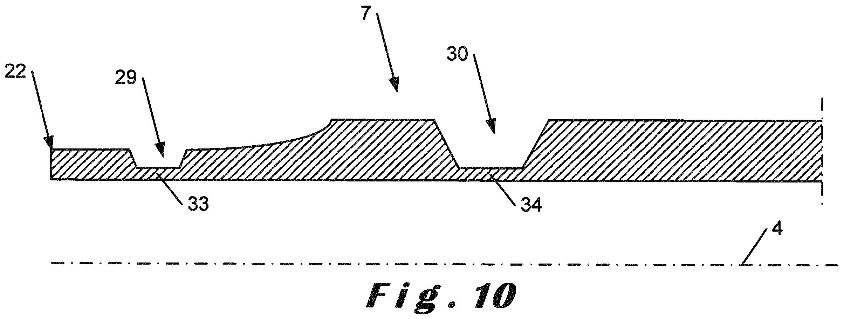

La figure 10 montre un autre mode réalisation où le canon 7 comprend un premier 29 et un deuxième 30 trous non traversant. Dans ce cas, il existe une première 33 et une deuxième 34 membranes, de préférence d'épaisseur comprise entre 10 et 100 µm, empêchant une communication directe entre l'intérieur et l'extérieur du canon quand il n'y a pas de surpression à l'intérieur du canon 7. Lorsqu'une surpression suffisante est atteinte dans le canon, ces membranes (33,34) se déchirent, ce qui permet alors à de l'air emprisonné entre le poussoir 3 et une lentille intraoculaire souple 10 de sortir. Utiliser des trous non débouchant délimités par des membranes au niveau de la paroi interne du canon 7 procure l'avantage suivant : le risque que la lentille intraoculaire souple 10 s'abîme au contact d'arrête vive est encore diminué voire annulé. Les dimensions géométriques des premier et deuxième (29 ;30) montrés à la figure 9A et présentées au paragraphe précédent peuvent s'appliquer aux trous (29 ;30) non traversant de la figure 10. De préférence, l'épaisseur des membranes (33,34) est comprise entre 20 et 40 µm.

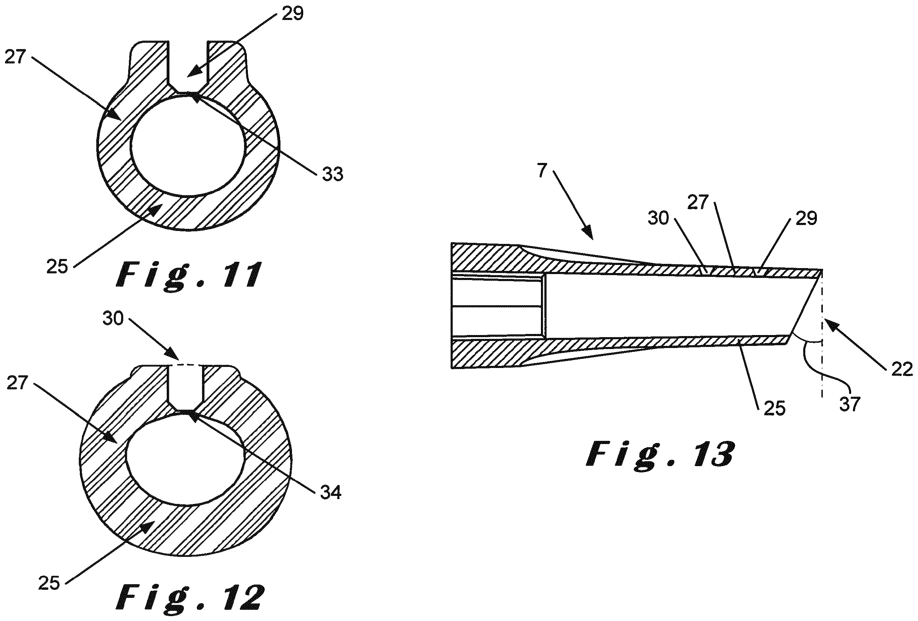

La figure 11 (respectivement 12) montre une section droite du canon 7 au niveau du premier trou 29 (respectivement deuxième trou 30), pour un mode de réalisation préféré de l'invention. Comme on peut le voir, il s'agit d'un trou non débouchant (ou non traversant) limité au niveau de la cavité d'éjection du canon 7 par une première membrane 33 (respectivement deuxième membrane 34). Comme on peut le voir sur cette figure le premier trou 29 (respectivement deuxième trou 30) n'a pas exactement la forme d'un cylindre ou d'un cône. De préférence, la première membrane 33 (respectivement deuxième membrane 34) est entourée d'un ou plusieurs chanfreins. Cela permet de limiter l'effet de bavures détachables ; ça limite donc les risques d'endommager une lentille intraoculaire souple 10 se déplaçant lors du percement de la ou des membranes (33, 34). Le ou les chanfreins relient la première membrane 33 (respectivement deuxième membrane 34) aux parois verticales du premier trou 29 (respectivement deuxième trou 30).

La figure 13 montre une forme préférée du canon 7 du dispositif d'injection de l'invention. Comme on peut le voir, le canon 7 est de préférence biseauté au niveau de l'orifice de sortie 22. La partie (de préférence moitié) supérieure 27 de la paroi latérale de canon s'étend davantage que la partie (de préférence moitié) inférieure 25 de la paroi latérale de canon parallèlement à la direction axiale 4. Dans ce cas, l'angle de biseau 37 défini entre une direction perpendiculaire à la direction axiale 4 et une section du canon 7 au niveau de l'orifice de sortie 22 est de préférence compris entre 20° et 30°, avec une valeur encore préférée de 25°. A la figure 13, le canon 7 comprend deux trous (29,30), mais il est envisageable de n'en prévoir qu'un ou d'en prévoir plus de deux avec un canon 7 biseauté.

La présente invention a été décrite en relation avec des modes de réalisations spécifiques, qui ont une valeur purement illustrative et ne doivent pas être considérés comme limitatifs. D'une manière générale, la présente invention n'est pas limitée aux exemples illustrés et/ou décrits ci-dessus. L'usage des verbes « comprendre », « inclure », « comporter », ou toute autre variante, ainsi que leurs conjugaisons, ne peut en aucune façon exclure la présence d'éléments autres que ceux mentionnés. L'usage de l'article indéfini « un », « une », ou de l'article défini « le », « la » ou « l' », pour introduire un élément n'exclut pas la présence d'une pluralité de ces éléments. Les numéros de référence dans les revendications ne limitent pas leur portée.

En résumé, l'invention peut également être décrite comme suit. Dispositif d'injection de lentille intraoculaire souple 10 comprenant :
- un corps tubulaire 1 présentant une cavité axiale ainsi qu'une extrémité proximale 5 et une extrémité distale 6,
- un poussoir 3 introduit à l'extrémité proximale 5, déplaçable dans cette cavité suivant une direction axiale 4,
- un canon 7 s'étendant au-delà de ladite extrémité distale 6, présentant une cavité d'éjection ayant une section transversale décroissante en sens opposé au corps tubulaire 1 et délimitée perpendiculairement à ladite direction axiale par une paroi latérale de canon, ledit canon 7 ayant un orifice de sortie 22,
la paroi latérale de canon comprenant un premier trou 29 traversant situé à une distance comprise 2 mm et 10 mm dudit orifice de sortie 22, de préférence entre 4 mm et 8 mm dudit orifice de sortie 22, et un deuxième trou (30) traversant situé à une distance comprise entre 6 mm et 10 mm dudit orifice de sortie (22).

## Revendications

1. Dispositif d'injection de lentille intraoculaire souple (10), comprenant :
- un corps tubulaire (1) présentant une enveloppe (2) et une cavité axiale ainsi qu'une extrémité proximale (5) et une extrémité distale (6),
- un poussoir (3) introduit dans ladite cavité axiale, à l'extrémité proximale (5) susdite, et déplaçable dans cette cavité suivant une direction axiale (4),
- un canon (7) s'étendant au-delà de ladite extrémité distale (6) du corps tubulaire (1), présentant une cavité d'éjection ayant une section transversale décroissante en sens opposé au corps tubulaire (1) et délimitée perpendiculairement à ladite direction axiale (4) par une paroi latérale de canon, ledit canon (7) ayant un orifice de sortie (22) pour permettre une sortie d'une lentille intraoculaire souple (10) en-dehors de ladite cavité d'éjection,
ladite paroi latérale de canon présentant une première partie (25) pour entrer en contact avec une lentille intraoculaire souple (10) repliée et une deuxième partie (27) pour entrer en moindre contact avec ladite lentille intraoculaire souple (10) repliée, quand ledit canon (7) contient ladite lentille intraoculaire souple (10),
**caractérisé en ce que**
ledit canon (7) comprend :
- un premier trou (29) traversant dans la deuxième partie (27) de sa paroi latérale de canon situé à une distance comprise entre 2 mm et 10 mm dudit orifice de sortie (22);
- un deuxième trou (30) traversant dans la deuxième partie (27) de sa paroi latérale de canon situé à une distance comprise entre 6 mm et 10 mm dudit orifice de sortie (22).

2. Dispositif d'injection selon la revendication précédente **caractérisé en ce que** la première partie (25) de la paroi latérale de canon est sans trou.

3. Dispositif d'injection selon l'une quelconque des revendications précédentes **caractérisé en ce qu'**il comprend en outre une navette (9) située entre le canon (7) et ladite extrémité proximale (5), ayant une cavité de stockage en communication avec ladite cavité d'éjection du canon (7) et la cavité axiale du corps tubulaire (1) pour recevoir une lentille intraoculaire souple (10), cette cavité de stockage étant délimitée perpendiculairement à ladite direction axiale (4) par une paroi latérale de navette,
ladite paroi latérale de navette présentant une première partie (26) pour entrer en contact avec une lentille intraoculaire souple (10) et une deuxième partie (28) pour entrer en moindre contact avec ladite lentille intraoculaire souple (10) quand ladite navette (9) comprend ladite lentille intraoculaire souple (10).

4. Dispositif d'injection selon la revendication précédente **caractérisé en ce que** ledit canon (7) et ladite navette (9) font partie d'une même pièce.

5. Dispositif d'injection selon la revendication 3 **caractérisé en ce que** ledit canon (7) et ladite navette (9) sont deux pièces séparées.

6. Dispositif d'injection selon l'une quelconque des revendications précédentes **caractérisé en ce que** :
- le premier trou (29) présente une symétrie de révolution autour d'un axe de premier trou qui est essentiellement perpendiculaire à ladite direction axiale (4) ; et/ou
- le deuxième trou (30) présente une symétrie de révolution autour d'un axe de deuxième trou qui est essentiellement perpendiculaire à ladite direction axiale (4).

7. Dispositif d'injection selon la revendication précédente **caractérisé en ce que** le premier trou (29) et/ou le deuxième trou (30) ont/a la forme d'un tronc de cône.

8. Dispositif d'injection selon la revendication 6 **caractérisé en ce que** le premier trou (29) et/ou le deuxième trou (30) ont/a une forme comprenant un ou plusieurs cylindres coaxiaux (301, 302, 303).

9. Dispositif d'injection selon la revendication précédente **caractérisé en ce que** les premier et deuxième trous (29, 30) ont une forme d'une superposition d'au moins deux cylindres (302, 303) comprenant un premier cylindre (302) d'un diamètre compris entre 0.7 et 0.5 mm, et un deuxième cylindre (303) d'un diamètre compris entre 0.4 et 0.2 mm, ledit premier cylindre (302) surmontant ledit deuxième cylindre (303).

10. Dispositif d'injection selon l'une quelconque des revendications précédentes **caractérisé en ce que** le premier trou (29) et/ou le deuxième trou (30) présentent/présente une extension minimale parallèlement à ladite direction axiale (4) comprise entre 0.3 mm et 0.5 mm.

11. Dispositif d'injection selon l'une quelconque des revendications précédentes **caractérisé en ce que** :
- ledit premier trou (29) est situé à 6 mm dudit orifice de sortie (22), et **en ce que**
- ledit deuxième trou (30) est situé à 8 mm dudit orifice de sortie (22).

12. Dispositif d'injection selon l'une quelconque des revendications précédentes **caractérisé en ce que**
- ledit canon (7) comprend une portion distale (71) et une portion proximale (72), ladite portion distale (71) comprenant ledit orifice de sortie (22), ladite portion proximale (72) étant comprise entre ladite portion distale (71) et ladite extrémité distale (6) du corps tubulaire (1); **en ce que**
- une épaisseur de ladite paroi latérale de canon de ladite portion distale (71), définie perpendiculairement à ladite direction axiale (4), est strictement plus petite qu'une épaisseur de ladite paroi latérale de ladite portion proximale (72) définie également perpendiculairement à ladite direction axiale (4) ; et **en ce que**
- lesdits premier (29) et deuxième (30) trous sont situés dans ladite portion proximale (72) dudit canon (7).

13. Dispositif d'injection selon l'une quelconque des revendications précédentes **caractérisé en ce que** le poussoir (3) est muni d'un manchon solidaire dudit poussoir (3) et/ou d'un coussin (40) en matière souple à son extrémité distale pour pousser une lentille intraoculaire souple (10).

14. Dispositif d'injection selon l'une quelconque des revendications précédentes comprenant une lentille intraoculaire souple (10).

## Patentansprüche

1. Vorrichtung zur Injektion einer biegsamen Intraokularlinse (10), umfassend:
- einen röhrenförmigen Körper (1) der eine Hülle (2) und einen axialen Hohlraum sowie ein proximales Ende (5) und ein distales Ende (6) aufweist;
- einen Stößel (3), der in den axialen Hohlraum an dem oben genannten proximalen Ende (5) eingeführt ist und in diesem Hohlraum in axialer Richtung (4) beweglich ist;
- eine Buchse (7), die sich über das distale Ende (6) des röhrenförmigen Körpers (1) hinaus erstreckt, einen Auswurfhohlraum aufweist, der in einer dem röhrenförmigen Körper (1) entgegengesetzten Richtung einen abnehmenden Querschnitt hat und senkrecht zu der axialen Richtung (4) durch eine Seitenwand der Buchse begrenzt ist, wobei die Buchse (7) eine Austrittsöffnung (22) hat, um einen Austritt einer biegsamen Intraokularlinse (10) aus dem Auswurfhohlraum zu ermöglichen,
wobei die Seitenwand der Buchse einen ersten Teil (25) zum Herstellen eines Kontakts mit einer gefalteten biegsamen Intraokularlinse (10) und einen zweiten Teil (27) zum Herstellen eines geringeren Kontakts mit der gefalteten biegsamen Intraokularlinse (10) aufweist, wenn die Buchse (7) die biegsame Intraokularlinse (10) enthält,
**dadurch gekennzeichnet, dass**
die Buchse (7) umfasst:
- ein erstes Loch (29), das durch den zweiten Teil (27) ihrer Seitenwand der Buchse verläuft, wobei es sich in einem Abstand zwischen 2 mm und 10 mm von der Austrittsöffnung (22) befindet;
- ein zweites Loch (30), das durch den zweiten Teil (27) ihrer Seitenwand der Buchse verläuft, wobei es sich in einem Abstand zwischen 6 mm und 10 mm von der Austrittsöffnung (22) befindet.

2. Vorrichtung zur Injektion nach dem vorstehenden Anspruch, **dadurch gekennzeichnet, dass** der erste Teil (25) der Seitenwand der Buchse ohne Loch ist.

3. Vorrichtung zur Injektion nach einem der vorstehenden Ansprüche, **dadurch gekennzeichnet, dass** sie weiter ein Schiffchen (9) umfasst, das zwischen der Buchse (7) und dem proximalen Ende (5) angeordnet ist, das einen Speicherhohlraum in Verbindung mit dem Auswurfhohlraum der Buchse (7) und dem axialen Hohlraum des röhrenförmigen Körpers (1) zur Aufnahme einer biegsamen Intraokularlinse (10) hat, wobei dieser Speicherhohlraum senkrecht zu der axialen Richtung (4) durch eine Seitenwand des Schiffchens begrenzt ist;
wobei die Seitenwand des Schiffchens einen ersten Teil (26) zum Herstellen eines Kontakts mit einer biegsamen Intraokularlinse (10) und einen zweiten Teil (28) zum Herstellen eines geringeren Kontakts mit der biegsamen Intraokularlinse (10) aufweist, wenn das Schiffchen (9) die biegsame Intraokularlinse (10) umfasst.

4. Vorrichtung zur Injektion nach dem vorstehenden Anspruch, **dadurch gekennzeichnet, dass** die Buchse (7) und das Schiffchen (9) zu demselben Teil gehören.

5. Vorrichtung zur Injektion nach Anspruch 3, **dadurch gekennzeichnet, dass** die Buchse (7) und das Schiffchen (9) zwei getrennte Teile sind.

6. Vorrichtung zur Injektion nach einem der vorstehenden Ansprüche, **dadurch gekennzeichnet, dass**
- das erste Loch (29) eine Rotationssymmetrie um eine Achse des ersten Lochs aufweist, die im Wesentlichen senkrecht zu der axialen Richtung (4) ist; und/oder
- das zweite Loch (30) eine Rotationssymmetrie um eine Achse des zweiten Lochs aufweist, die im Wesentlichen senkrecht zu der axialen Richtung (4) ist.

7. Vorrichtung zur Injektion nach dem vorstehenden Anspruch, **dadurch gekennzeichnet, dass** das erste Loch (29) und/oder das zweite Loch (30) die Form eines Kegelstumpfes haben/hat.

8. Vorrichtung zur Injektion nach Anspruch 6, **dadurch gekennzeichnet, dass** das erste Loch (29) und/oder das zweite Loch (30) eine Form haben/hat, die einen oder mehrere koaxiale Zylinder (301, 302, 303) umfasst.

9. Vorrichtung zur Injektion nach dem vorstehenden Anspruch, **dadurch gekennzeichnet, dass** das erste und das zweite Loch (29, 30) die Form einer Überlagerung von mindestens zwei Zylindern (302, 303) haben, die einen ersten Zylinder (302) mit einem Durchmesser zwischen 0,7 und 0,5 mm und einen zweiten Zylinder (303) mit einem Durchmesser zwischen 0,4 und 0,2 mm umfassen, wobei der erste Zylinder (302) den zweiten Zylinder (303) überragt.

10. Vorrichtung zur Injektion nach einem der vorstehenden Ansprüche, **dadurch gekennzeichnet, dass** das erste Loch (29) und/oder das zweite Loch (30) eine minimale Ausdehnung parallel zu der axialen Richtung (4) zwischen 0,3 mm und 0,5 mm aufweisen/aufweist.

11. Vorrichtung zur Injektion nach einem der vorstehenden Ansprüche, **dadurch gekennzeichnet, dass**
- sich das erste Loch (29) 6 mm von der Austrittsöffnung (22) entfernt befindet, und dass
- sich das zweite Loch (30) 8 mm von der Austrittsöffnung (22) entfernt befindet.

12. Vorrichtung zur Injektion nach einem der vorstehenden Ansprüche, **dadurch gekennzeichnet, dass**
- die Buchse (7) einen distalen Abschnitt (71) und einen proximalen Abschnitt (72) umfasst, wobei der distale Abschnitt (71) die Austrittsöffnung (22) umfasst, wobei der proximale Abschnitt (72) zwischen dem distalen Abschnitt (71) und dem distalen Ende (6) des röhrenförmigen Körpers (1) liegt; dass
- eine Dicke der Seitenwand der Buchse des distalen Abschnitts (71), die senkrecht zu der axialen Richtung (4) definiert ist, streng kleiner als eine Dicke der Seitenwand des proximalen Abschnitts (72) ist, die ebenfalls senkrecht zu der axialen Richtung (4) definiert ist; und dass
- sich das erste (29) und das zweite (30) Loch in dem proximalen Abschnitt (72) der Buchse (7) befinden.

13. Vorrichtung zur Injektion nach einem der vorstehenden Ansprüche, **dadurch gekennzeichnet, dass** der Stößel (3) mit einer Hülse, die mit dem Stößel (3) fest verbunden ist, und/oder mit einem Polster (40) aus biegsamem Material an seinem distalen Ende versehen ist, um eine biegsame Intraokularlinse (10) zu schieben.

14. Vorrichtung zur Injektion nach einem der vorstehenden Ansprüche, umfassend eine biegsame Intraokularlinse (10).

## Claims

1. Device for injecting a flexible intraocular lens (10), comprising:
- a tubular body (1) having a casing (2) and an axial cavity as well as a proximal end (5) and a distal end (6),
- a plunger (3) inserted into said axial cavity, at the aforementioned proximal end (5), and capable of being moved in this cavity in an axial direction (4),
- a barrel (7) extending beyond said distal end (6) of the tubular body (1), having an ejection cavity that has a decreasing cross-section away from the tubular body (1) and delimited perpendicularly to said axial direction (4) by a side wall of the barrel, said barrel (7) having an outlet opening (22) to allow for an exiting of a flexible intraocular lens (10) outside of said ejection cavity,
said side wall of the barrel having a first portion (25) to come into contact with a folded flexible intraocular lens (10) and a second portion (27) to come into least contact with said folded flexible intraocular lens (10), when said barrel (7) contains said flexible intraocular lens (10),
**characterised in that** said barrel (7) comprises:
- a first through-hole (29) in the second portion (27) of its side wall of the barrel located at a distance of between 2 mm and 10 mm from said outlet opening (22);
- a second through-hole (30) in the second portion (27) of its side wall of the barrel located at a distance of between 6 mm and 10 mm from said outlet opening (22).

2. Device for injecting according to the preceding claim, **characterised in that** the first portion (25) of the side wall of the barrel does not have any hole.

3. Device for injecting according to any one of the preceding claims, **characterised in that** it further comprises a shuttle (9) located between the barrel (7) and said proximal end (5), having a storage cavity in communication with said ejection cavity of the barrel (7) and the axial cavity of the tubular body (1) to receive a flexible intraocular lens (10), this storage cavity being delimited perpendicularly to said axial direction (4) by a side wall of the shuttle, said side wall of the shuttle having a first portion (26) to come into contact with a flexible intraocular lens (10) and a second portion (28) to come into least contact with said flexible intraocular lens (10) when said shuttle (9) comprises said flexible intraocular lens (10).

4. Device for injecting according to the preceding claim, **characterised in that** said barrel (7) and said shuttle (9) form part of one same part.

5. Device for injecting according to claim 3, **characterised in that** said barrel (7) and said shuttle (9) are two separate parts.

6. Device for injecting according to any one of the preceding claims, **characterised in that**:
- the first hole (29) has a symmetry of revolution about an axis of the first hole which is substantially perpendicular to said axial direction (4); and/or
- the second hole (30) has a symmetry of revolution about an axis of the second hole which is substantially perpendicular to said axial direction (4).

7. Device for injecting according to the preceding claim, **characterised in that** the first hole (29) and/or the second hole (30) have/has the shape of a truncated cone.

8. Device for injecting according to claim 6, **characterised in that** the first hole (29) and/or the second hole (30) have/has a shape of comprising one or more coaxial cylinders (301, 302, 303).

9. Device for injecting according to the preceding claim, **characterised in that** the first and second holes (29, 30) have a shape of a superposition of at least two cylinders (302, 303) comprising a first cylinder (302) of a diameter between 0.7 and 0.5 mm, and a second cylinder (303) of a diameter between 0.4 and 0.2 mm, said first cylinder (302) surmounting said second cylinder (303).

10. Device for injecting according to any one of the preceding claims, **characterised in that** the first hole (29) and/or the second hole (30) has/have a minimum extension parallel to said axial direction (4) between 0.3 mm and 0.5 mm.

11. Device for injecting according to any one of the preceding claims, **characterised in that**:
- said first hole (29) is located at 6 mm from said outlet opening (22), and **in that**
- said second hole (30) is located at 8 mm from said outlet opening (22).

12. Device for injecting according to any one of the preceding claims, **characterised in that**:
- said barrel (7) comprises a distal portion (71) and a proximal portion (72), said distal portion (71) comprising said outlet opening (22), said proximal portion (72) being comprised between said distal portion (71) and said distal end (6) of the tubular body (1); **in that**
- a thickness of said side wall of the barrel of said distal portion (71), defined perpendicularly to said axial direction (4), is strictly smaller than a thickness of said side wall of said proximal portion (72) also defined perpendicularly to said axial direction (4); and **in that**
- said first (29) and second (30) holes are located in said proximal portion (72) of said barrel (7).

13. Device for injecting according to any one of the preceding claims, **characterised in that** the plunger (3) is provided with a sleeve integral with said plunger (3) and/or a pad (40) made of flexible material at its distal end to push a flexible intraocular lens (10).

14. Device for injecting according to any one of the preceding claims, comprising a flexible intraocular lens (10).
